# EUROPÄISCHE PATENTSCHRIFT

(11) **EP 1 446 164 B1**
(45) Veröffentlichungstag und Bekanntmachung des Hinweises auf die Patenterteilung: **30.03.2005**
(21) Anmeldenummer: 02790382.2
(22) Anmeldetag: 15.11.2002
(51) Int. Cl.: A61L 2/00, A23C 9/142, A01J 11/06, A23J 1/20

(54) **VERFAHREN ZUR REDUZIERUNG DER GESAMTKEIMZAHL IN WÄSSRIGEN DISPERSIONEN NICHT-HOMOGENER ZWEI- ODER MEHRPHASENGEMISCHE**
METHOD FOR REDUCING THE TOTAL BACTERIA COUNT IN AQUEOUS DISPERSIONS OF NON-HOMOGENEOUS TWO-PHASE OR MULTI-PHASE MIXTURES
PROCEDE DE REDUCTION DE L'INDICE DE GERMINATION TOTAL DANS DES DISPERSIONS AQUEUSES DE MELANGES NON HOMOGENES A DEUX PHASES OU A PHASES MULTIPLES

(30) Priorität: 21.11.2001 DE 10158009
(43) Veröffentlichungstag der Anmeldung: 18.08.2004
(73) Patentinhaber: E.Begerow Gmbh & Co, 55450 Langenlonsheim (DE)
(72) Erfinder: GEIGER, Günther, 55593 Rüdesheim (DE); TESCH, Ralf, 55131 Mainz (DE)
(74) Vertreter: Bartels, Martin Erich Arthur
(86) Internationale Anmeldenummer: PCT/EP2002/012794
(87) Internationale Veröffentlichungsnummer: WO 2003/043664

(56) Entgegenhaltungen:
- EP-A- 0 798 003
- DE-A- 4 026 365
- GB-A- 1 573 995
- US-A- 5 707 678
- US-B1- 6 288 222
- HOU K ET AL: "CAPTURE OF LATEX BEADS, BACTERIA, ENDOTOXIN AND VIRUSES BY CHARGE- MODIFIED FILTERS" APPLIED AND ENVIRONMENTAL MICROBIOLOGY, WASHINGTON,DC, US, Bd. 40, Nr. 5, November 1980 (1980-11), Seiten 892-896, XP001053164 ISSN: 0099-2240

## Beschreibung

Die Erfindung betrifft ein Verfahren zur Reduzierung der Gesamtkeimzahl mit den Merkmalen des Oberbegriffes des Patentanspruches 1.

In einem im Jahr 2000 veröffentlichten Fachaufsatz "Entwicklungstendenzen bei der Käseherstellung" (H.-P. Bachmann, H. Schär, R. Sieber, H. Winkler, F. Rentsch) der eidgenössichen Forschungsanstalt für Milchwirtschaft, Schweiz, werden unter Ziffer 3 "Neue Technologien bei der Käseherstellung" vorgestellt. Dabei wird herausgestellt, daß bei der neuen Technologie für die Käseherstellung die Behandlung der Milch im Vordergrund steht. Dabei geht es auf der einen Seite darum, die Milch möglichst keimarm zu machen und auf der anderen Seite solle die chemische Zusammensetzung der Milch gezielt verändert werden.

Als Verfahren zur Entkeimung der Milch werden neben den bekannten verschiedenen Wärmebehandlungsverfahren (Thermisation, Pasteurisation) die Mikrofiltration vorgestellt unter Einsatz von Trennmembranen, wobei aufgrund des Porendurchmessers der Membran von 1,4 µm alle Mehrmagermilchkomponenten die Membran passieren und nur die Bakterien und Sporen zurückgehalten werden. Da die Bakterien und die Fettkügelchen ungefähr dieselbe Größe aufweisen, muß bei der Membranfiltration das Fett vor der Mikrofiltration abzentrifugiert werden und nur die Magermilch wird mikrofiltriert. Das anfallende Retentat (Bakterrenkonzentrat) und der Rahm werden mit einer ultrahocherhitzenden Temperaturanlage bei 130°C während 4 Sekunden hocherhitzt und anschließend der Fabrikationsmilch wieder zugegeben. Damit kann eine weitgehend sporenfreie Milch verarbeitet werden. Es hat sich unter Einsatz einer Pilotanlage gezeigt, mit einer Kapazität von 500 l Milch pro Stunde, daß der Konzentrationsfaktor 20:1 (5% Retentat) beträgt und daß die Buttersäurebakterien und Sporen bei der Mikrofiltration mittels Trennmembranen mit einem sehr hohen Wirkungsgrad abgetrennt werden, wobei auch die Zahl der übrigen Bakterien vermindert ist.

Neben den genannten Membrantrennverfähren ist durch den vorstehend bezeichneten Fachaufsatz auch die Entkeimungsseparation bekannt, wobei die sogenannte Baktofugation das bekannteste Verfahren zur Keimreduktion ist. Bei dem dahingehenden seit mehr als 20 Jahren bekannten Verfahren wird der Effekt ausgenützt, daß die Sporen spezifisch schwerer sind als die Milch, wobei um den Wirkungsgrad zu verbessern, die Entkeimungsseparation häufig zweimal nacheinander durchgeführt wird. Weitere Entkeimungsverfahren stellen sogenannte Hochdruckverfahren dar, bei denen die Behandlung der Milch mit hydrostatischem Druck von 1000 bis 10 000 bar für zwei bis 60 Minuten bei 20 bis 30 °C erfolgt. Dabei werden insbesondere die vegetativen Mikroorganismen durch den Druck auf ihre Membran wirkungsvoll abgetötet. Sporen und Enzyme sind aber weitgehend resistent. Zudem werden die Kaseinmicellen und die Molkenproteine nachteilig verändert.

Darüber hinaus ist die Ultrafiltration als weiteres Membrantrennverfahren bekannt, bei der mit Hilfe eines Druckgefälles hochmolekulare von niedermolekularen Stoffen abgetrennt und aufkonzentriert werden. Bei der Ultrafiltration von Magermilch werden die Proteine (Kaseine, Molkeinproteine) der Milch durch die Membran im Retentat zurückgehalten, während Salze, Lactose und niedermolekulare Stickstoffverbindungen der Membran in das Permeat passieren. Mit zunehmenden Konzentrationsgrad steigen demzufolge die Trockenmasse und der Proteingehalt im UF-Retentat, das Verhältnis Kasein zu Molkenprotein bleibt jedoch unverändert. Das dahingehende Ultrafiltrations-Membrantrennverfahren erlaubt in einer Vorbehändlungsstufe der Milch die Erhöhung des Einbaus von nativen Molkenproteinen, was die Qualität der Milch günstig beeinflußt.

Die vorstehend bezeichneten Sterilisations- und Entkeimungsverfahren sind insbesondere, sofern sie Trennmembranen einsetzen, verfahrenstechnisch teuer in der Anwendung, wozu auch der hohe Preis der einzusetzenden Membranen mit beiträgt und das Milchfett (Lipide) ist regelmäßig vor dem eigentlichen Trennverfahren abzuscheiden, um das Zusetzen der Membran, was man auch mit Verblocken bezeichnet, zu verhindern. Für einen großtechnischen Einsatz mit hohen Mengen an wäßrigen Dispersionen, wie Milch für die Sterilfiltration, ist mithin nicht zu denken.

Um die Prozeßverfahrensschritte im großtechnischen Maßstab zu verbessern und insbesondere einen vollautomatischen Betriebsablauf zu gewähren, sind unter der Markenbezeichnung TETRA ALCROSS ™ MB Mikrofiltrationsanlagen mit Keramik-Membranen der Firma Tetra Pak im Einsatz, die gleichfalls auf eine Mikrofiltration mittels. Trennmembranen zurückgreifen, die jedoch im vorliegenden Fall als Keramikmembranen aus Aluminiumoxyd und/oder anderen Metalloxyden ausgebildet sind mit einer Porengröße zwischen 0,1 bis 1,4 Mikron. Aufgrund des Einsatzes der Keramikmembranen sind diese mit Heißwasser sterilisierbar, hochbelastbar, chemisch widerstandsfähig in einem weiten pH-Bereich und weisen hohe Berstdrücke auf. Die Kriterien zur Auswahl der Keramikmembran bildet die Rückhalterate für Einweiß und gewünschte Effekte der Eiweißfraktionierung, z.B. Trennung von Kasein und Molkenprotein sowie die Abtrennung von Mikroorganismen. Die eingesetzten Keramikmembranen erzeugen dabei entlang der gesamten Membranfläche einen niedrigen, konstanten und gleichförmigen Transmembrandruck, was einen hohen und konstanten Durchsatz erlaubt. Mit den dahingehenden Mikrofiltrationssystemen läßt sich ein gattungsgemäßes Verfahren durchführen, wobei ein vollautomatischer Betriebsablauf erreichbar ist mit hohen Durchsatzmengen. Aufgrund des Einsatzes von Keramikmembranen als Trennmembranen ist jedoch auch das bekannte Verfahren teuer und aufwendig in der Realisierung. Ferner neigen die Keramikmembranen gleichfalls zur Verblockung.

Durch die PCT-WO 96/32021 ist ein gattungsgemäßes Verfahren bekannt, das jedoch nur die Keimreduzierung von Milchserum erlaubt, d.h. lediglich von der kontinuierlichen Phase einer Dispersion. Mithin bezieht sich das bekannte Verfahren nach der PCT - Veröffentlichung auf die Filtration einer homogenen wäßrigen Lösung, wobei als Tiefenfiltereinrichtung geladene (polare) Tiefenfilter mit ihren Filter-Medien eingesetzt werden. Mit dem dahingehend bekannten Verfahren unter Einsatz der geladenen Tiefenfiltermedien wäre die Entkeimung von zwei Phasensystemen in der Art einer wäßrigen Flüssig-Flüssig-Dispersion als Zwei-Phasengemisch, wie Milch, nicht möglich und sollte man in diesem Zusammenhang, was die PCT - Veröffentlichung nahelegt, Filterhilfsmittel einsetzen, wäre mit deren sofortigem Verblocken und somit Unbrauchbarwerden zu rechnen.

Ein ähnliches Verfahren ist auch Gegenstand der EP-A-0 798 003, das die Titer-Reduktion von Viren in einer wäßrigen Lösung gleichfalls unter Einsatz von elektrisch geladenen Tiefenfiltermedien erlaubt. Auch mit der dahingehenden Lösung ist die Keimreduktion eines nicht homogenen Zwei- oder Mehr-Phasengemisches, wie Milch oder Magermilch, nicht möglich. Würde man bei der bekannten Lösung die polaren (geladenen) Filtermedien bei Zwei-Phasengemischen einsetzen, würde es bei einer statischen Filtration zu einer sofortigen Verblockung des Filtermediums kommen.

Durch die DE 40 26 365 A ist eine sterilfiltrierte, caseinhaltige Kolostral-Milch und ein Verfahren zu ihrer Herstellung bekannt. Die Kolostral-Milch wird auf einen pH-Wert von 2,5 - 3,5 eingestellt und dann klärfiltriert und sterilfiltriert. Vorzugsweise wird die Kolostral-Milch vor dieser Behandlung entfettet und nach der Säurebehandlung vor den Filtrationsmaßnahrnen auf einen pH-Wert von 5,5 - 8 eingestellt. Bei der bekannten Lösung wurde nun überraschenderweise gefunden, dass man Kololstral-Mitch klärfiltrieren und sterilfiltrieren kann, ohne vorher die Casein-Fraktion zu entfernen, wenn man die gegebenenfalls entfettete Kolostral-Milch auf einen-pH-Wert von unter 3,5 ansäuert. Beim Herabsenken des pH-Wertes fällt zwar das Casein bei pH 4 bis 5 aus, wird aber durch das weitere Absenken des pH-Wertes wieder in Lösung gebracht. Diese saure Lösung ist in ihren Eigenschaften gegenüber der Kolostral-Milch soweit verändert, dass sie nun sterilfiltrierbar ist.

Zwar läßt die bekannte Lösung offen, ob der beispielsweise für die Klärfiltrierung der Kolostral-Magermilch eingesetzte Tiefenfilter polar oder unpolar ausgestaltet ist. Durch den Fachaufsatz von HOU K ET AL: "CAPTURE OF LATEX BEADS, BACTERIA, ENDOTOXIN AND VIRUSES BY CHARGE-MODIFIED FILTERS" APPLIED AND ENVIRONMENTAL MICROBIOLOGY, WASHINGTON, DC, US, Bd.40, Nr.5, November 1980 (1980-11), Seiten 892-896, XP001053164 ISSN: 0099-2240, ist es aber bekannt, dass sich die Ladung des Filters zwangsweise bei niederen pH-Werten ändert, so dass der gegebenenfalls unpolar verwendete Tiefenfilter nach der vorstehend genannten Entgegenhaltung (DE 40 26 365 A) jedenfalls während des Einsatzes polar wird.
Bei der genannten Offenbarung nach HOU K et al geht es im wesentlichen um die Abscheidung von Viren aus wäßrigen Lösungen, wobei Latexkugelnur zur Simulation der Viren verwendet werden, um wissentschaftlich präzise Partikeldurchmesser zu haben. Die Latexergebnisse sind somit auf Viren übertragbar. Bei der bekannten Lösung wird unter anderem ein Versuch dargestellt mit ungeladenem Filter, bei dem nur noch der sog. "Siebeffekt" des Filters wirksam ist. HOU K et al beschreibt den allgemeinen Zusammenhang von Ladung des Filters und der Ladung der zu filtrierenden Bestandteile. Je nachdem, was in der wäßrigen Disperson vorliegt, ist eine jeweils andere Ladung oder keine Ladung des Filters sinnvoll, um ein vorschnelles Blockieren des Filters zu vermeiden. Die Ladung spielt jedoch nur dann eine Rolle, wenn es um die Abscheidung von Partikeln mit einem Partikeldurchmesser kleiner dem "Siebeffekt" des Filters geht. Während sich also HOU K et al mit der Reduzierung der Gesamtkeimzahl in wäßrigen Dispersonen beschäftigt, die dem Siebeffekt des Filters unterliegen, sind bereits aufgrund der Komplexität der Systeme Milch sowie Milchprodukte die erhaltenen Ergebnisse dahingehend nicht übertragbar.

Ausgehend von diesem Stand der Technik liegt der Erfindung die Aufgabe zugrunde, die bekannten Verfahren dahingehend weiter zu verbessern, dass auf kostengünstige Weise und mit hohen Durchsatzmengen in großtechnischem Maßstab eine effiziente Reduzierung der Gesamtkeimzahl in wäßrigen Dispersonen (Zwei- und Mehrphasensysteme) durch Sterilfiltration möglich ist unter Vermeidung von Verblockungen an den Filtereinrichtungen und von Vorabscheidungen von Lipiden und/oder Proteinen vor der eigentlichen Sterilfiltration. Eine dahingehende Aufgabe löst ein Verfahren mit den Merkmalen des Patentanspruches 1 in seiner Gesamtheit.

Dadurch, dass gemäß dem kennzeichnenden Teil des Patentanspruches 1 als wäßrige Dispersionen nicht-homogene Zwei- oder Mehr-Phasengemische eingesetzt werden, nämlich in Form tierischer Milch und daraus gewonnenen Produkten, wie Magermilch mit vorgebbarem Gehalt an Dispergenzien, insbesondere Lipiden und Proteinen, und daß die Tiefenfilter (18,22) der jeweiligen Tiefenfiltereinrichtung elektrisch ungeladen (unpolar) sind, daß die Reduzierung der Gesamtkeimzahl mit einer Effektivität erfolgt, die einem logarithmischen Keimrückhaltewert (LRV-Wert) von 2 bis 5 entspricht, daß die Filtermedien mit einer nominalen Abscheiderate von < als 1µm, insbesondere < als 0,5 µm, vorzugsweise zwischen 0,2 und 0,3 µm, versehen werden, und daß die Temperatur der jeweiligen zu filtrierenden Dispersion <als 60°C, vorzugsweise 40°C bis 50°C, jedoch> als 10°C gewählt wird, ist eine Sterilfiltration von Dispersonen in Form von tierischer Milch erreicht, und zwar in großtechnischem Maßstab mit hohen Durchsatzmengen.

Da die Tiefenfiltereinrichtungen eine fortlaufende Filtration erlauben und keine Aufspaltung in Retentat und Permeat erfolgt und insbesondere berücksichtigend, daß die Tiefenfiltermedien resistenter sind als die bekannten Membranschichten, lassen sich sehr hohe Differenzdrücke (zwischen 0 bis 5 bar) einhalten, was maßgebend zur Erhöhung der zu behandelnden Durchflußmenge führt.

Da darüber hinaus die Filtermedien bei den Tiefenfiltereinrichtungen unpolar (elektrisch ungeladen) sind, treten Veiblockungen wie bei den ansonsten bekannten eingesetzten Trennfiltermembranen und Tiefenfiltereinrichtungen nicht auf und es ist auch nicht notwendig, die Protein- und/oder Lipidteile der wäßrigen Dispersion vorher abzuscheiden, um zu guten Sterilfiltrationsergebnissen zu gelangen. Die Tiefenfiltereinrichtungen lassen sich in einer Vielzahl von Ausführungsformen in großseriellem Maßstab herstellen und sind so relativ kostengünstig zu erhalten, was wiederum die Kosten des Gesamtverfahrens zur Reduzierung der Gesamtkeimzahl in wäßrigen Dispersionen deutlich senkt. Sofern die Filter der jeweiligen Tiefenfiltereinrichtung zugesetzt sind, lassen diese sich in einfacher und kostengünstiger Weise rückspülen und somit immer wieder regenerieren, so daß die Vorrichtung zum Durchführen des erfindungsgemäßen Verfahrens weitgehend produktionstechnischen Charakter erhält und nicht den eines Versuchsaufbaus unter Einsatz der Membranfilterschichten. Insbesondere gegenüber den Keramiktrennfiltermedien sind die Kosten für den Einsatz von Tiefenfiltereinrichtungen deutlich reduziert.

Es ist für einen Fachmann auf dem Gebiet der Sterilfiltration überraschend, daß er durch Einsatz von Tiefenfiltereinrichtungen mit Tiefenfiltern die deutlich kostengünstiger zu erhalten sind, als die bekannten Trennfltermembranen, eine deutlich verbesserte Entkeimung erhalten kann, bei hohen Durchsatzmengen an Unfiltrat und daß dabei gleichzeitig der Lipid- und/oder Proteingehalt der Dispersion durch die Tiefenfiltration im wesentlichen unverändert bleibt, insbesondere nicht nachteilig beeinflußt wird. In praktischen Versuchen hat sich dabei gezeigt, daß die Gesamtkeimzahl des Filtrats mit dem erfindungsgemäßen Verfahren < als 200 koloniebildenden Einheiten pro Milliliter (KBE/ml) ist.

Weitere vorteilhafte Ausführungsformen sind Gegenstand der sonstigen Unteransprüche.

Im folgenden wird das erfindungsgemäße Verfahren anhand einer Vorrichtung zu ihrem Durchführen nach der Zeichnung näher erläutert. Dabei zeigen in prinzipieller und nicht maßstäblicher Darstellung die
- Fig. 1: den grundsätzlichen Aufbau einer Filtrationsanlage zum Durchführen des erfindungsgemäßen Verfahrens,
- Fig. 2: in perspektivischer Ansicht eine Tiefenfilterkerze als Filter für die Vorrichtung nach der Fig. 1.

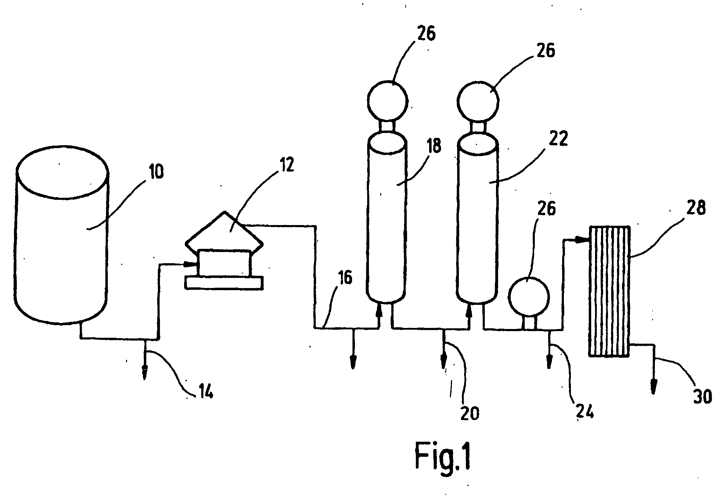

Das erfindungsgemäße Verfahren zur Reduzierung der Gesamtkeimzahl (GKZ) in wäßrigen Dispersionen erfolgt durch Sterilfiltration besagter Dispersionen. Eine dahingehende Dispersion kann beispielsweise in Form einer Suspension tierischer Milch und daraus gewonnener Produkte vorliegen, wie Magermilch mit vorgebbaren Gehalt an Dispergenzien, insbesondere in Form von Lipiden und Proteinen. Aufgabe des erfindungsgemäßen Verfahrens ist es, die Gesamtkeimzahl zu reduzieren, und zwar durch ein Tiefenfiltrationsverfahren, ohne daß der Lipid- und/oder Proteingehalt sich für das Produkt nachteilig ändert. Die Fig. 1 zeigt den grundsätzlichen Aufbau einer Vorrichtung zum Durchführen des Verfahrens, insbesondere in Form der Sterilfiltration in der Art eines Ablaufschemas. Die in einem Rohmilchtank 10 befindliche Rohmilch ist einem Seperator 12 zuführbar, wobei vor dem Seperator 12 eine Abzweigmöglichkeit 14 für die Rohmilch besteht. In Fluidrichtung hinter dem Seperator 12 läßt sich an einer weiteren Abzweigstelle 16 Magermilch erhalten, die ansonsten einem Filter 18 zuführbar ist, an den sich seriell hinter einer ersten Filtratentnahmestelle 20 ein zweiter Filter 22 anschließt, der ausgangsseitig in eine Fluidleitung mündet mit einer weiteren Filtratentnahmestelle 24. Über Manometer oder sonst druckerfassende Sensoren 26 läßt sich die Drucksituation im Filtrationskreis erfassen. Am Ausgang des zweiten Filters 22 kann sich eine Pasteurisierungseinrichtung 28 anschließen, sofern man neben sterilfiltrierter Milch am Ausgang 30 pasteurisierte Milch erhalten will.

Vor Beginn der Filtration werden zunächst die beiden Filter 18 und 22 mit Dampf sterilisiert. Desweiteren werden die beiden Filter 18, 22 zusammen mit der kompletten Sterilfiltrationsanlage mit Heißwasser angefahren und erst danach auf Rohmilch umgestellt. Der benötigte Filtrationsdruck wurde dabei ausschließlich durch den Seperator 12 erzeugt. Beim automatischen "entschlammen" fällt der Druck bis auf 0 bar Differenzdruck ab und baut sich nach dem Abreinigen unmittelbar wider auf. Beim Anfahren und Nachspülen der beiden Filter 18, 22 entsteht kein Anfangsdruckverlust.

Die Sterilfiltration der Rohmilch wird mittels Tiefenfiltereinrichtungen vorgenommen und die Filter 18, 22 sind demgemäß als Tiefenfilter ausgebildet, insbesondere in der Art von Tiefenfilterkerzen. Bei der Tiefenfiltration durchströmendes Unfiltrat von innen nach außen, im vorliegenden Fall jedoch bevorzugt von außen nach innen, die jeweilige Tiefenfilterkerze 18, 22 und das Filtrat wird aus dem Inneren des jeweiligen Filters 18 bzw. 22 zur Weiterverwendung abgeführt. Bei der Tiefenfiltration findet also eine permanente Filtrierung statt und die eingesetzten Tiefenfilterkerzen erlauben hohe Durchflußraten an filtrierendem Medium (Unfiltrat).

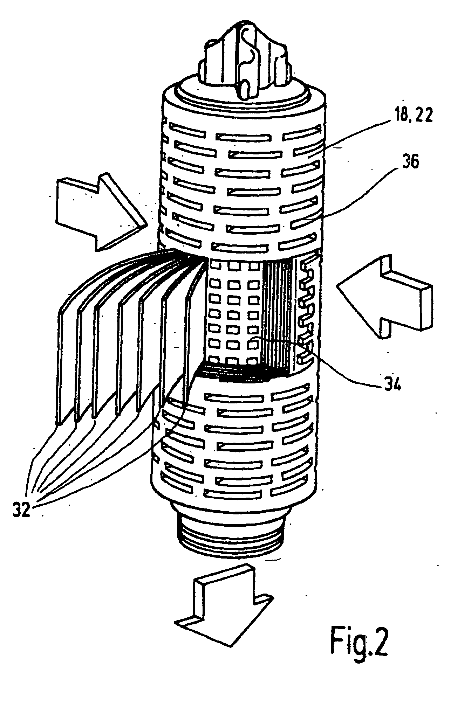

Mit der gezeigten Tiefenfiltereinrichtung nach Fig. 1 erfolgt die Reduzierung der Gesamtkeimzahl (GKZ) mit einer Effektivität, die einem logarithmischen Keim-Rückhaltewert (LRV-Wert) von 2 bis 5 entspricht, wobei der dahingehende logarithmische Wert aus dem Verhältnis von Gesamtkeimzahl Filtrat zu Gesamtkeimzahl Unfiltrat erhalten wird. Bei der in Fig. 2 gezeigten zweistufigen Filtration mit zwei in Linie hintereinandergeschalteten Tiefenfiltern 18 bzw. 22 lassen sich durchaus LRV-Werte bis 4,2 erreichen. Dies entspricht einer Keimreduzierung auf < als 100 Keimen pro Milliliter. Es hat sich dabei ferner gezeigt, daß durch den Einsatz der Tiefenfilter 18, 22 weder Fette (Lipide) noch Eiweiße (Proteine) abgetrennt wurden, so daß die qualitätsbildenden Faktoren der Milch erhalten bleiben.

Die bei den Tiefenfiltern 18, 22 eingesetzten Filtermedien sind solche mit einer nominalen Abscheiderate von 0,2 bis 0,3 µm. Ferner werden Temperaturen für die zu filtrierende Dispersion zwischen 40°C bis 50°C gewählt, was zu guten Keimreduzierungszahlen führt. Ferner ist im angesprochenen Temperaturbereich erreicht, daß das in der Milch befindliche Kasein und/oder Fett die Tiefenfilter 18, 22 nicht verblocken und damit zumindest teilweise unbrauchbar machen kann. Als besonders geeignet haben sich Tiefenfilter erwiesen, die im Produktprogramm der Anmelder unter BECO-PROTECT KTB 373 mit 0,3 µm nominaler Abscheiderate geführt sind, sowie BECO-PROTECT KTB 273 mit einer nominalen Abscheiderate von 0,2 µm. Hierbei haben sich ohne weiteres logarithmische Keimrückhaltewerte von 0,8 bis 2,3 bzw. von 1,6 bis 2 ergeben. Die dahingehenden Werte ergeben sich bei Anströmgeschwindigkeiten von 300 l/Std. für eine 30-Zoll-Tiefenfilterkerze, so daß die Anströmgeschwindigkeit auf die Fläche der Kerze selbst ca. V = 1200 l pro m² und Stunde ist. Sehr gute Werte haben sich auch ergeben, sofern man für den zweistufigen Filtrationsprozeß anstelle von Rohmilch Magermilch einsetzt.

Die bei der Milchfiltration zum Einsatz kommenden Tiefenfilterkerzen mit nominalen Abscheideraten zwischen 0,2 und 0,3 µm bestehen vorzugsweise aus Polypropylen-Meltblown-Lagen 32, wie sie in der Fig. 2 dargestellt sind. Eine einzelne Tiefenfilterkerze kann dabei durchaus bis zu acht verschiedenen Meltblown-Vliese 32 übereinander enthalten. Von außen nach innen gesehen baut sich die Filterkerze zunächst aus offenen Meltblown-Vliesen auf, die unkalandriert sind, wobei weiter dem Inneren zu sukzessiv Meltblown-Lagen verwendet werden, die eine dichtere Geometrie aufweisen, wobei die dichteren Geometrien dadurch erreicht sind, daß die Meltblown-Vliese bei ihrer Herstellung kalandriert werden, d.h. beispielsweise mittels Kalanderwalzen eine Verdichtung erfahren.

Die genannten Meltblown-Vlieslagen 32 werden um ein inneres Stützrohr 32 gewickelt, das mit Perforationen versehen dem Ablauf des Filtrats aus der Tiefenfilterkerze 18, 22 dient. Zum Schutz der Meltblown-Vlieslagen 32 nach außen hin weist die jeweilige Tiefenfilterkerze 18, 22 außenumfangsseitig einen Mantel 36 auf, der gleichfalls mit Perforierungen, beispielsweise in Form von Längsschlitzen versehen ist, und dergestalt den Eintritt des Unfiltrats in die jeweilige Filterkerze 18, 22 erlaubt. Die Durchströmungsrichtung des Unfiltrats ist in Fig. 2 mit zwei gegenläufigen Pfeilen dargestellt und an der Unterseite der Tiefenfilterkerze ist der Austritt des Filtrats mit einem einfachen Pfeil wiedergegeben. Der dahingehende außenliegende Stützmantel 36 kann bei bestimmten Bauarten von Filterkerzen auch entfallen.

Wie die Entkeimung der Rohmilch über die Sterilfiltration läßt sich die dahingehende Filtration auch bei Magermilch ohne weiteres durchführen, wobei dann mit einer Keimbelastung < 100, vorzugsweise < 10 koloniebildenden Einheiten pro Milliliter Filtrat gerechnet werden kann. So lassen sich mit 30 Zoll Polypropylen-Tiefenfilterkerzen bei einem Volumenstrom von ca. 300 I/Std. Magermilch, die Keime in der Magermilch um mindestens ca. 99 % reduzieren. Die wesentlichen Bestandteile der Milch, wie Proteine und Lipide, bleiben dabei trotzt der Filtration erhalten. Die Sterilfiltration mittels Tiefenfiltereinrichtung erlaubt hohe Durchflußraten und läßt sich sehr kostengünstig realisieren. Das Verfahren ist also insbesondere auch geeignet für die Sterilfiltration von Molke.

Im folgenden wird das erfindungsgemäße Verfahren noch anhand zweier Anwendungsbeispiele näher erläutert:

### Beispiel 1: filtration von Magermilch.

Magermilch hat erfahrungsgemäß noch einen Restfettgehalt von ca. 0,1 % und ist somit eine sehr verdünnte Öl/Wasser-Emulsion (Zwei-Phasengemisch). Bei Vorversuchen mit polaren (geladenen) Filtermedien (sowohl Tiefenfiltermedien aus Zellulose als auch Membranfilter (0,45 µm)) wird bei einer statischen Filtration eine sofortige Verblockung des Filtermediums beobachtet.

Bei mehreren Versuchen bezogen auf das erfindungsgemäße Verfahren wurde eine zweistufige Filtrationsanlage in eine vorhandene Milchbearbeitungslinie integriert. Es wurde ein Teilstrom der Magermilch vom Separator kommend statisch filtriert. Die Temperatur der Magermilch beträgt 42°C. Die Filtration wird über eine Zeitdauer von 200 Minuten durchgeführt bei kontinuierlicher Erfassung des Durchsatzes und des Druckanstieges während der Filtration. Während des Versuches wurden im Abstand von 60 Minuten mikrobiologische Proben vor und nach der Filtereinheit entnommen.

### Mikrobiologische Auswertung:

Die Bestimmung der Gesamtkeimzahl in den Proben wurde nach dem Keimzählverfahren nach Koch (Methodenbuch Band IV, M 6.3.1, 1985 mit 4. Ergänzungslieferung 1996) durchgeführt. Zur Bestimmung der thermoduren (hitzeresistenten) Keime wurden die Proben im Wasserband für 30 Minuten auf 65°C erhitzt und anschließend abgekühlt, weitere Handhabung wie Keimzählverfahren nach Koch. Psychotrophe Keime (sich in der Kälte ernährend) wurden wiederum nach dem Keimzählverfahren nach Koch angesetzt, jedoch für 7 Tage bei 10°C bebrütet.

### Ergebnisse:

Während der Versuchszeit wurde nur ein geringer Druckanstieg von 0,3 bar an der jeweiligen Tiefenfiltereinheit beobachtet. Die Keimreduktionsraten sind in folgender Tabelle zusammengefaßt:

| **Gesamtkeimzahl** | | | |
|---|---|---|---|
| Zeit: [min] | GKZ vor Filter [KBE/ml] | GKZ nach Filter [KBE/ml] | Keimreduktion: LRV (logarithmic reduction value) [-] |
| 20 | 27.455 | 20 | 3,14 |
| 80 | 26.410 | 80 | 2,52 |
| 140 | 28.500 | 165 | 2,24 |
| 200 | 16.640 | 185 | 1,95 |

| **Coliforme Keime** | | | |
|---|---|---|---|
| Zeit: [min] | Coliforme Keime vor Filter [KBE/ml] | Coliforme Keime nach Filter [KBE/ml] | Keimreduktion: LRV (logarithmic reduction value) [-] |
| 20 | 80 | 0 | 100 |
| 80 | 50 | 0 | 100 |
| 140 | 500 | 0 | 100 |
| 200 | 260 | 4 | 99,4 |

| **Thermodure Keime** | | | |
|---|---|---|---|
| Zeit: [min] | Thermodure Keime vor Filter [KBE/ml] | Thermodure Keime nach Filter [KBE/ml] | Keimreduktion: [%] |
| 20 | 131 | 0 | 100 |
| 80 | 111 | 0 | 100 |
| 140 | 131 | 0 | 100 |
| 200 | 157 | 0 | 100 |

| **Psychrotrophe Keime** | | | |
|---|---|---|---|
| Zeit: [min] | GKZ vor Filter [KBE/ml] | GKZ nach Filter [KBE/ml] | Keimreduktion: [%] |
| 20 | 2.505 | 1 | 100 |
| 80 | > 3000 | 6 | 99,8 |
| 140 | > 3000 | 25 | 99,2 |
| 200 | 4250 | 23 | 99,5 |

Während des Versuches wurde keine Abreicherung des Fettgehaltes der Milch beobachtet. (Fettgehalt der Magermilch ca. 0,1 %).
Das durchgeführte Verfahren ergab, daß bei allen untersuchten Keimklassen gemäß den vorstehenden Übersichten eine Reduktion größer 99% erzielt wurde. Da insbesondere die thermophilen und die psychotrophen Keime für den Verderb von Frischmilch verantwortlich sind, konnten diese mit dem beschriebenen Verfahren erfolgreich aus der Magermilch abgetrennt werden.

### Beispiel 2: Filtration von Vollmilch.

Im laufenden Prozeß einer Milchverarbeitungslinie wurde nach dem Separator ein Vollmilchstrom von 1000 l/h abgezweigt und statisch filtriert. Diese teilstromhomogenisierte Vollmilch hat einen Fettanteil von 3,5 %, der als ölige Phase in der Öl/Wasser-Emulsion (Zwei-Phasengemisch) vorliegt. Aufgrund der Größe der Fetttröpfchen wird jeder polare Tiefenfilter und jede übliche Filtrationsmembran (z.B. 0,45 µm) bei statischer Filtration gemäß den bekannten Verfahren sofort verblocken.

Die Filtrationstemperatur lag bei 55°C. Der Homogenisierungsdruck betrug 170 bar bei einstufiger Homogenisierung. Während der Versuchszeit wurde der Durchfluß konstant gehalten und der Druckanstieg am Filter aufgezeichnet. Im 30 Minuten Abstand wurden mikrobiologische Proben gezogen und gemäß dem vorstehend genannten Beispiel untersucht.

### Ergebnisse:

Die Druckdifferenz bei der Filtration betrug am Ende der Filtrationszeit von 140 min lediglich 0,2 bar.

Ergebnisse der mikrobiologischen Untersuchungen:

| **Gesamtkeimzahl** | | | |
|---|---|---|---|
| Zeit: [min] | GKZ vor Filter [KBE/ml] | GKZ nach Filter [KBE/ml] | Keimreduktion: LRV (logarithmic reduction value) [-] |
| 15 | 6.400 | 885 | 0,86 |
| 45 | 7.850 | 775 | 1,01 |
| 75 | 6.800 | 465 | 1,17 |
| 105 | 9.091 | 1.186 | 0,88 |
| 140 | 10.136 | 973 | 1,02 |

| **Coliforme Keime** | | | |
|---|---|---|---|
| Zeit: [min] | Coliforme Keime vor Filter [KBE/ml] | Coliforme Keime nach Filter [KBE/ml] | Keimreduktion: [%] |
| 15 | 50 | 9 | 82 |
| 45 | 75 | 11 | 85,3 |
| 75 | 60 | 2 | 96,7 |
| 105 | 15 | 6 | 60 |
| 140 | 10 | 5 | 50 |

| **Thermodure Keime** | | | |
|---|---|---|---|
| Zeit: [min] | Thermodure Keime vor Filter [KBE/ml] | Thermodure Keime nach Filter [KBE/ml] | Keimreduktion: [%] |
| 15 | 1560 | 89 | 94,3 |
| 45 | 1625 | 126 | 92,2 |
| 75 | 870 | 89 | 89,9 |
| 105 | 1155 | 126 | 89,1 |
| 140 | 1340 | 65 | 95,1 |

| **Psychrotrophe Keime** | | | |
|---|---|---|---|
| Zeit: [min] | GKZ vor Filter [KBE/ml] | GKZ nach Filter [KBE/ml] | Keimreduktion: [%] |
| 15 | 2.195 | 23 | 96,5 |
| 45 | 10.500 | 11 | 98,7 |
| 75 | 11.300 | 1 | 99,9 |
| 105 | 13.100 | 20 | 96,3 |
| 140 | 11.850 | 13 | 97,4 |

Die erhaltenen Ergebnisse zeigen, daß die teilstromhomogenisierte Vollmilch mit Keimreduktionswerten von durchschnittlich 90% entkeimt wurde. Durch einen nachgeschalteten Pasteurisationsschritt kann eine Vollmilch mit Keimzahlen kleiner 10 BE/ml erhalten werden. Die Haltbarkeit einer solchen Vollmilch verlängert sich dadurch, ohne daß die Nachteile der sonst üblichen Pasteurisierung zum Tragen kämen.

Mit dem erfindungsgemäßen Verfahren ist eine Dead-End-Filtration durchführbar, die gegenüber den bekannten Filtrationsverfahren, auch gegenüber der bekannten Crossflow-Filtration unter anderem den Vorteil bietet, daß die Filter oder Filterelemente zu ihrer Regenerierung rückspülbar sind und im übrigen elektrisch unpolar sind, was die Bereitstellungskosten für die Filterelemente reduzieren hilft.

## Patentansprüche

1. Verfahren zur Reduzierung der Gesamtkeimzahl in wäßrigen Dispersionen durch Sterilfiltration besagter Dispersionen mittels Tiefenfilter (18,22) mindestens einer Tiefenfiltereinrichtung unter vorgebbaren Bedingungen, insbesondere der Temperatur des Unfiltrats, dem Differenzdruck an den Tiefenfiltern (18, 22) und der Anströmgeschwindigkeit des Unfiltrats auf die jeweilige Tiefenfiltereinrichtung, **dadurch gekennzeichnet, daß** als wäßrige Dispersionen nicht-homogene Zwei- oder Mehr-Phasengemische eingesetzt werden, nämlich in Form tierischer Milch und daraus gewonnenen Produkten, wie Magermilch mit vorgebbarem Gehalt an Dispergenzien, insbesondere Lipiden und Proteinen, und daß die Tiefenfilter (18,22) der jeweiligen Tiefenfiltereinrichtung elektrisch ungeladen (unpolar) sind, daß die Reduzierung der Gesamtkeimzahl mit einer Effektivität erfolgt, die einem logarithmischen Keimrückhaltewert (LRV-Wert) von 2 bis 5 entspricht, daß die Filtermedien mit einer nominalen Abscheiderate von < als 1µm, insbesondere < als 0,5 µm, vorzugsweise zwischen 0,2 und 0,3 µm, versehen werden, und daß die Temperatur der jeweiligen zu filtrierenden Dispersion < als 60°C, vorzugsweise 40°C bis 50°C, jedoch> als 10°C gewählt wird.

2. Verfahren nach Anspruch 1, **dadurch gekennzeichnet, daß** der Lipid- und/oder Proteingehalt der Dispersion durch die Tiefenfiltration im wesentlichen unverändert bleibt.

3. Verfahren nach Anspruch 1 oder 2, **dadurch gekennzeichnet, daß** die Gesamtkeimzahl des Filtrats < als 1000 koloniebildenden Einheiten pro Milliliter (KBE/ml), insbesondere < als 200 KBE/ml, vorzugsweise < als 10 KBE/ml gehalten wird.

4. Verfahren nach einem der Ansprüche 1 bis 3, **dadurch gekennzeichnet, daß** die Anströmgeschwindigkeit auf die Filter (18, 22) < als 1500 l pro m² und Stunde, insbesondere 500 bis 1200 l pro m² und Stunde, jedoch > 300 l pro m² und Stunde eingestellt wird.

5. Verfahren nach einem der Ansprüche 1 bis 4, **dadurch gekennzeichnet, daß** der Differenzdruck an den Filtern (18, 22) zwischen 0 bar und 5 bar gehalten wird.

6. Verfahren nach einem Ansprüche 1 bis 5, **dadurch gekennzeichnet, daß** die Tiefenfiltereinrichtung mit mehreren Filterlinien betrieben wird, die jeweils mit mindestens einem Tiefenfilter (18, 22) bestückt werden.

7. Verfahren nach einem der Ansprüche 1 bis 6, **dadurch gekennzeichnet, daß** die Tiefenfilter (18, 22) mit vorgebbaren, insbesondere unterschiedlicher nominaler Abscheiderate eingesetzt werden.

8. Verfahren nach einem der Ansprüche 1 bis 7, **dadurch gekennzeichnet, daß** als Tiefenfilter Filterkerzen eingesetzt werden.

9. Verfahren nach einem der Ansprüche 1 bis 8, **dadurch gekennzeichnet, daß** als Material für die Filtermedien Polypropylen verwendet wird.

10. Verfahren nach einem der Ansprüche 1 bis 9, **dadurch gekennzeichnet, daß** als Filtermedien Vliese, insbesondere nach dem Meltblown-Verfahren gewonnene Vliese (32) eingesetzt werden.

## Claims

1. Method for the reducing of the total germ count in aqueous dispersions through the sterile filtration of said dispersion with a depth filter (18, 22) of at least one depth filter means under pre-deterrninable conditions, especially the temperature of the unfiltrate, the pressure differential at the depth filters (18, 22), and the streaming speed of the unfiltrate towards the relevant depth filter means, **characterised in that** the utilised aqueous dispersion are non-homogeneous two- or multi-phase mixtures, namely in the form of animal milk and products derived from the same, such as low-fat milk with a pre-determinable dispergent content, especially lipids and proteins, and **in that** the depth filters (18, 22) of the relevant depth filter means are electrically uncharged (non-polar), **in that** the reducing of the total germ count is carried out with an effectiveness which equals a logarithmic germ retention value (LRV value) of 2 to 5, and **in that** the filter media are equipped with a nominal precipitation rate of < than 1 µm, especially < than 0.5 µm, and preferably between 0.2 and 0.3 µm, and **in that** the temperature of the relevant dispersion that is to be filtered is < then 60°C, preferably 40°C to 50°C, not > than 10°C.

2. Method according to Claim 1, **characterised in that** the lipid and/or protein content of the dispersion remains substantially unchanged by the depth filtration.

3. Method according to Claim 1 or 2, **characterised in that** the total germ count of the filtrate is kept to < than 1000 colony-forming units per millilitre (KBE/ml), especially < than 200 KBE/ml, preferably < than 10 KBE/ml.

4. Method according to one of the Claims 1 to 3, **characterised in that** the streaming speed towards the filters (18, 22) is adjusted to < than 1500l per m² and hour, especially 500 to 1200l per m² and hour, but > than 300l per m² and hour.

5. Method according to one of the Claims 1 to 4, **characterised in that** the pressure difference at the filters (18, 22) is maintained at between 0 bar and 5 bar.

6. Method according to one of the Claims 1 to 5, **characterised in that** the depth filter means is operated with several filter lines which are each equipped with at least one depth filter (18, 22).

7. Method according to one of the Claims 1 to 6, **characterised in that** the depth filters (18, 22) are operated with pre-determinable, especially different nominal precipitation rates.

8. Method according to one of the Claims 1 to 7, **characterised in that** filter candles are utilised as depth filters.

9. Method according to one of the Claims 1 to 8, **characterised in that** polypropylene is used as a material for the filter media.

10. Method according to one of the Claims 1 to 9, **characterised in that** fleece, especially fleece (32) produced with the aid of the melt blown process, is used as a filter media.

## Revendications

1. Procédé de réduction du nombre total de germes dans les dispersions aqueuses par filtration stérile desdites dispersions au moyen de filtres à lit profond (18,22), d'au moins un dispositif à filtres à lit profond dans des conditions spécifiables, en particulier de température du non-filtré, de pression différentielle sur les filtres à lit profond (18,22) et de vitesse de filtration du non-filtré sur le dispositif de filtration à lit profond considéré, **caractérisé en ce que** des mélanges non homogènes bi- ou polyphasiques sont utilisés comme dispersions aqueuses, à savoir sous forme de lait animal et de produits qui en sont dérivés tels que le lait écrémé avec une teneur spécifiable en dispersants, notamment en lipides et protéines, et **en ce que** les filtres à lit profond (18,22) des dispositifs de filtration à lit profond concernés ne sont pas électriquement chargés (non polaires), que la réduction du nombre total de germes s'effectue avec une efficacité correspondant à une valeur logarithmique de retenue des germes de 2 à 5, que les médias de filtration sont dotés d'un taux de séparation nominal de moins de 1 µm, notamment de moins de 0,5 µm, de préférence entre 0,2 et 0,3 µm, et que la température de la dispersion à filtrer est inférieure à 60°C, de préférence à 40°C à 50°C mais supérieure à 10°C.

2. Procédé selon la revendication 1, **caractérisé en ce que** la teneur en lipides et/ou protéines de la dispersion reste en grande partie inchangée par la filtration sur lit profond.

3. Procédé selon la revendication 1 ou 2, **caractérisé en ce que** le nombre total de germes du filtrat est maintenu inférieur à 1 000 unités formant des colonies par millilitre (KBE/ml), notamment inférieur à 200 KBE/ml, de préférence inférieur à 10 KBE/ml.

4. Procédé selon l'une quelconque des revendications 1 à 3, **caractérisé en ce que** la vitesse de filtration sur les filtres (18,22) est inférieure à 1 500 l par m² et par heure, notamment 500 à 1 200 l par m² et par heure, mais supérieure à 300 l par m² et par heure.

5. Procédé selon l'une quelconque des revendications 1 à 4, **caractérisé en ce que** la pression différentielle sur les filtres (18,22) est maintenue entre 0 et 5 bar.

6. Procédé selon l'une quelconque des revendications 1 à 5, **caractérisé en ce que** le dispositif à filtres à lit profond est exploité avec plusieurs lignes de filtration chacune équipée d'au moins un filtre à lit profond (18,22).

7. Procédé selon l'une quelconque des revendications 1 à 6, **caractérisé en ce que** le filtre à lit profond (18,22) peut être ajusté à plusieurs taux de séparation nominaux spécifiables, notamment différents.

8. Procédé selon l'une quelconque des revendications 1 à 7, **caractérisé en ce que** des bougies filtrantes sont utilisées comme filtre à lit profond.

9. Procédé selon l'une quelconque des revendications 1 à 8, **caractérisé en ce que** du polypropylène est employé comme matériau pour les médias filtrants.

10. Procédé selon l'une quelconque des revendications 1 à 9, **caractérisé en ce que** des non-tissés, notamment des non-tissés (32) obtenus par le procédé de fusion-soufflage, sont utilisés comme médias filtrants.
